# FASCICULE DE BREVET EUROPEEN

(11) **EP 0 475 844 B1**
(45) Date de publication et mention de la délivrance du brevet: **17.04.1996**
(21) Numéro de dépôt: 91402422.9
(22) Date de dépôt: 12.09.1991
(51) Int. Cl.: A61K 31/55

(54) **Utilisation de N-(1-hexahydroazepinylalkyl) acétamides dans la préparation des médicaments destinés au traitement des troubles de la transmission cholinergique**
Verwendung von N)1-hexahydroazepinyl) acetamiden zur Herstellung von Arzneimitteln zur Behandlung von Strörungen des cholinergen Transmittersystems
Use of N-(1.hexahydroazepinylalkyl) acetamides for the manufacture of medicaments for the treatment of disorders of the cholineogic transmission

(30) Priorité: 12.09.1990 FR 9011266
(43) Date de publication de la demande: 18.03.1992
(73) Titulaire: UNIVERSITE DE CAEN, F-14032 Caen (FR)
(72) Inventeur: Israel, Maurice, F-91440 Bures sur Yvette (FR); Morot-Gaudry, Yvette, F-91190 Gif sur Yvette (FR); Robba, Max, F-75004 Paris (FR); Adam, Gérard, F-78600 Le Mesnil le Roi (FR); Renard, Pierre, F-78000 Versailles (FR)
(74) Mandataire: Kedinger, Jean-Paul

(56) Documents cités:
- DE-A- 2 313 338
- W. FORTH et al.: "Allgemeine und Spezielle Pharmakologie und Toxikologie", édition 5, 1987, pages 103-121, Bi Wissenschaftsverlag, Mannheim, DE; "Pharmakologische Beeinflussung der cholinergen Erregungsübertragung"

## Description

La présente invention concerne l'utilisation de N - (1 - hexahydroazépinylalkyl) acétamides dans la préparation de médicaments destinés au traitement des troubles de la transmission cholinergique.

On connaît de l'art antérieur, notamment par le brevet allemand DE 2313338, des N - (1 - hexahydroazépinylalkyl) acétamides dotés de propriétés spasmolytiques ou vasodilatatrices.

La demanderesse a maintenant découvert que de tels N - (1 - hexahydroazépinylalkyl) acétamides possédaient la propriété surprenante, d'agir sur la transmission cholinergique en stimulant puissamment la libération d'acétylcholine.

Cette activité a pour conséquence une augmentation de la concentration en acétylcholine au niveau de la synapse cholinergique et de la jonction neuromusculaire et elle trouve son application dans les pathologies dues à un déficit cholinergique central ou périphérique, comme la maladie d'Alzheimer, certains déficits intellectuels dus à la sénescence, ou la myasthénie.

En effet, l'altération de la transmission de l'influx nerveux au niveau des synapses cholinergiques du système nerveux central constitue une des causes physiologiques de la symptomatologie associée à la maladie d'Alzheimer (Johns C.A. et al "The cholinergic treatment strategy in aging and senile dementia". Psychopharmacol. Bull - 1983, 19, 185-187 ; Collerton D "Cholinergic function and intellectual decline in Alzheimer's disease". Neuroscience 1986, 19, 1 - 28) et des troubles cognitifs et intellectuels de la sénéscence (Davidson M et al "Cholinergic mechanisms in the treatment of geriatric disorders" Psychopharmacol - Bull, 1986, 22, 101 - 105).

Quelques uns des traitements actuellement proposés de la maladie d'Alzheimer reposent d'ailleurs sur une stimulation du système cholinergique.
- soit de façon directe comme par l'administration d'agents para sympathomimétiques (arécoline),
- soit de façon indirecte par des précurseurs de l'acétylcholine (choline, lécithine) augmentant la production d'acétylcholine au niveau synaptique, ou par des inhibiteurs d'acétylcholinesterase (physostigmine) réduisant la dégradation enzymatique du neuromédiateur.

Ces thérapeutiques se heurtent toutefois à des problèmes liés à une demi-vie courte du principe actif et à un rétro-contrôle diminuant la production d'acétylcholine dans le neurone.

La myasthénie se caractérise quant à elle par un blocage post-synaptique de la jonction neuro-musculaire, zone de contact entre les motoneurones et le muscle effecteur (Engel A.G. et al "Histometric analysis of the ultrastructure of the neuromuscular junction in myasthenia gravis and the myasthenic syndrome". Ann. N.Y. Acad. Sci, 1976, 183, 46 - 63). Le neuromédiateur responsable de la transmission de l'influx nerveux au niveau de la jonction neuromusculaire est l'acétylcholine. Si la myasthenie a vraisemblablement une origine auto-immune, sa traduction physiologique réside dans un blocage de la transmission cholinergique et les seules médications ayant montré une activité dans le traitement de cette maladie sont des inhibiteurs d'acétylcholinestérase, mais leur emploi est toutefois limité par la fréquence des effets secondaires qu'ils engendrent.

Les substances comme les dérives selon l'invention stimulant la libération d'acétylcholine physiologique trouvent donc une application naturelle notamment dans le traitement de la maladie d'Alzheimer et des troubles apparentes, de la myasthénie, et également de toute autre affection centrale ou périphérique résultant d'un déficit de la transmission cholinergique et l'on peut attendre d'une stimulation de libération naturelle qu'elle n'entraine pas d'effets secondaires.

Plus spécifiquement, l'invention concerne l'utilisation, dans la préparation de médicaments destinés au traitement des troubles de la transmission cholinergique, de N - (1 - hexahydroazépinylalkyl) acétamides de formule générale I. 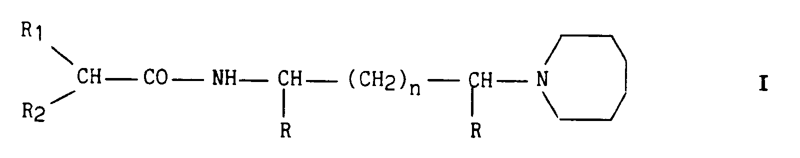
dans laquelle :
- R représente un atome d'hydrogène ou un radical alkyle de 1 à 4 atomes de carbone,
- n représente 0, 1, 2, 3 ou 4,
- R₁ et R₂, identiques ou différents représentent
   . un groupement alkyle ou alkényle renfermant de 1 à 6 atomes de carbone, en chaine droite ou ramifiée,
   . un groupement cycloalkyle comportant de 3 à 7 atomes de carbone ou cycloalkényle comportant de 5 à 7 atomes de carbone,
   . un radical aryle, aralkyle, héteroaryle ou hétéroaralkyle éventuellement substitué pour un ou plusieurs groupements choisis parmi halogène, alkoxy, nitro, trifluoromethyle ou hydroxyalkyle,
leurs isomères ainsi que leurs sels d'addition à un acide pharmaceutiquement acceptable.

On entend par aryle les radicaux phenyle ou naphtyle et par héteroaryle les radicaux furyle, thiényle, pyrrolyle, imidazolyle, benzofuryle, benzothiényle, benzimidazolyle, indolyle.

Comme acide pharmaceutiquement acceptable permettant de salifier les composés de l'invention on peut citer, à titre non limitatif les acides chlorhydrique, bromhydrique, sulfurique, nitrique, oxalique, fumarique, tartrique, méthanesulfonique, éthanesulfonique, camphorique, camphosulfonique, citrique, malique, maléique,...

Les médicaments, obtenus en utilisant, selon l'invention, un composé de formule I, l'un de ses isomères ou l'un de ses sels pharmaceutiquement acceptables, sont présentes avantageusement sous des formes diverses telles que par exemple comprimés, dragées, gélules, glossettes ou autres préparations galéniques appropriées pour une administration sublinguale, suppositoires, solutions pour pulvérisation endobuccale, préparations galéniques appropriées pour une administration percutanée, solutions injectables ou buvables.

La posologie peut varier largement en fonction de l'âge du patient, de son poids, la voie et la fréquence d'administration, la gravité de l'affection dont souffre le patient et des traitements associes et s'échelonne de 0,05 à 150 mg par prise ou par application.

L'invention est illustrée, au titre non limitatif par l'activité sur la transmission cholinergique des composés suivants :
- - compose A :: N - [3 - (1 - hexahydroazépinyl) propyl] diphenylacetamide, chlorhydrate,
- - composé B :: N - [2 - ( 1 - hexahydroazépinyl ) éthyl ] diphénylacétamide, chlorhydrate,
- - compose C :: cyclohexyl N - [3 - (1 - hexahydroazepinyl) propyl] thien - 3 ylacétamide, chlorhydrate,
- - compose D :: cyclohexyl N - [3 - (1 - hexahydroazépinyl) propyl] benzo [b] thién - 2 ylacétamide, chlorhydrate.

Les tests rapportés sont réalisés sur l'organe électrique de Torpille (Torpedo marmorata), dont il est reconnu qu'il est embryologiquement homologue des jonctions neuromusculaires cholinergiques des vertèbres supérieurs.

### EXAMPLE 1 :

### STIMULATION DE LA LIBERATION D'ACETYLCHOLINE IN VITRO

Les terminaisons nerveuses cholinergiques de l'organe électrique de Torpille sont isolées selon la technique décrite par M. ISRAEL (Biochem. J. 1976, 160, 113 - 115). Les synaptosomes sont purifiés sur un gradient discontinu de densité de mélanges isoosmotiques saccharose - NaCl proches du sérum de Torpille, composé de 250 mM de NaCl , 3 mM KCl, 1,8 mM MgCl₂, 3,4 mM CaCl₂, 5mM NaHCO₃, 5,5 mM glucose, 300 mM urée et 100 mM saccharose. Le pH est porté à une valeur de 7,1 avec 1,2 mM de tampon phosphate Na, après équilibration avec O₂.

Les terminaisons ainsi préparées sont capables de synthétiser de l'acétylcholine radioactive à partir de précurseurs marqués (choline et acétate), elles sont riches en acétylcholine qu'elles sont capables de libérer après stimulation.

La libération d'acétylcholine est provoquée, en présence de 3,5 mM de calcium par addition d'un ionophore à calcium, le A23187 à la concentration de 3,5 µM, qui s'insère dans la membrane des synaptosomes et permet par échange électroneutre entre le calcium extracellulaire et les cations intracellulaires, une augmentation de la concentration calcique cytoplasmique et la libération du neuromédiateur.

De façon pratique, des volumes de 50 µl de la préparation de synaptosomes, correspondant à 30 mg de tissu, sont dilués dans 450 µl d'une solution alcaline proche du sérum de Torpille (voir ci-dessus). L'application des composés à tester a lieu 3 à 5 minutes dans ce milieu avant que la libération d'acétylcholine soit provoquée.

Le dosage de l'acétylcholine est effectué selon la technique de chimiluminescence décrite par ISRAEL et LESBATS (J. Neurochem, 1981, 37, 1475 et Neurochem Int, 1981, 3, 81). L'émission lumineuse qui se produit pendant quelques minutes est calibrée immédiatement après par comparaison avec un standard interne d'acétylcholine. A la fin de l'expérience de libération, l'addition du Triton X-100 (0,05 %) provoque la rupture des membranes synaptosomales permettant ainsi la détermination de l'acétylcholine encore occluse dans les synaptosomes de l'échantillon.

Les composés utiles selon l'invention possèdent la propriété de stimuler la libération d'acétylcholine quand celle-ci est induite par une augmentation du calcium intracellulaire par addition de l'ionophore à calcium A 23187 en présence de calcium, comme indiqué dans le tableau ci-après :

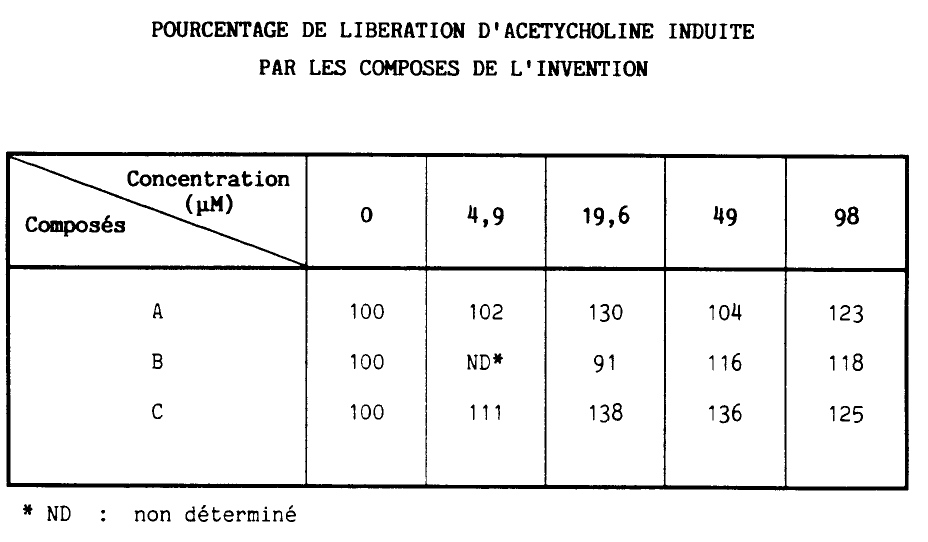

### EXEMPLE 2 : Soluté injectable dosé à 0,1 mg/ml de chlorhydrate de cyclohexyl N - [3 - (1 - hexahydroazépinyl) propyl] thién - 3 - ylacétamide

| | |
|---|---|
| Cyclohexyl N - [3 - (1 - hexahydroazépinyl) propyl] thién - 3 - ylacétamide, chlorhydrate | 0,2 g |
| . eau pour préparation injectable qsp | 2 l |
| (quantités pour 1 000 ampoules injectables dosées à 0,2 mg de principe actif). | |

### EXEMPLE 3 : Gélules dosées à 1 mg de chlorhydrate de cyclohexyl N - [3 - (1 - hexahydroazépinyl) propyl] benzo [b] thién - 2 ylacétamide

| | |
|---|---|
| Cyclohexyl N - [3 - (1 - hexahydroazépinyl) propyl] benzo [b] thien - 2 ylacétamide, Chlorhydrate | 1 g |
| . Stéarate de magnésium | 5 g |
| . Lactose | 20 g |
| . Silice colloïdale | 1 g |
| (quantités pour 1 000 gélules N° 2 dosées à 1 mg de principe actif). | |

## Revendications (Revendications pour l'(les) Etat(s) contractant(s) suivant(s): AT, BE, CH, DE, DK, FR, GB, IT, LI, LU, NL, SE)

1. Utilisation d'un composé de formule I : 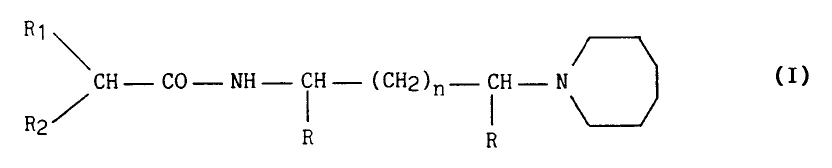 dans laquelle :
- R représente un atome d'hydrogène ou un radical alkyle de 1 à 4 atomes de carbone,
- n représente 0, 1, 2, 3, ou 4,
- R₁ et R₂, identiques ou différents représentent :
. un groupement alkyle ou alkényle renfermant de 1 à 6 atomes de carbone, en chaine droite ou ramifiée,
. un groupement cycloalkyle comportant de 3 à 7 atomes de carbone ou cycloalkényle comportant de 5 à 7 atomes de carbone,
. un radical aryle, aralkyle, hétéroaryle ou hétéroaralkyle éventuellement substitué pour un ou plusieurs groupements choisis parmi halogène, alkoxy, nitro, trifluorométhyle ou hydroxyalkyle, étant entendu que par aryle on désigne les radicaux phényle ou naphtyle et par hétéroaryle on désigne les radicaux furyle, thiényle, pyrrolyle, imidazolyle, benzofuryle, benzothiényle, benzimidazolyle, indolyle,
l'un de ses isomères ainsi que l'un de ses sels d'addition à un acide pharmaceutiquement acceptable, pour l'obtention de médicaments utiles dans le traitement des troubles de la transmission cholinergique.

2. Utilisation selon la revendication 1 de N - [3 - (1 - hexahydroazépinyl) propyl] diphénylacétamide, un de ses isomères ou un de ses sels d'addition à un acide pharmaceutiquement acceptable pour l'obtention de médicaments utiles dans le traitement des troubles de la transmission cholinergique.

3. Utilisation selon la revendication 1 de N - [2 - (1 - hexahydroazépinyl) éthyl] diphénylacétamide, un de ses isomères ou un de ses sels d'addition à un acide pharmaceutiquement acceptable pour l'obtention de médicaments utiles dans le traitement des troubles de la transmission cholinergique.

4. Utilisation selon la revendication 1 de Cyclohexyl N - [3 - (1 - hexahydroazépinyl) propyl] thién - 3 ylacétamide, un de ses isomères ou un de ses sels d'addition à un acide pharmaceutiquement acceptable pour l'obtention de médicaments utiles dans le traitement des troubles de la transmission cholinergique.

5. Utilisation selon la revendication 1 de Cyclohexyl N - [3 - (1 - hexahydroazépinyl) propyl] benzo [b] thién - 2 ylacétamide, un de ses isomères ou un de ses sels d'addition à un acide pharmaceutiquement acceptable pour l'obtention de médicaments utiles dans le traitement des troubles de la transmission cholinergique.

6. Utilisation d'un composé selon l'une des revendications 1 à 5 pour l'obtention de médicaments utiles dans le traitement de la maladie d'Alzheimer, des désordres cérébraux liés à la sénescence ou des myasthénies.

## Revendications (Revendications pour l'(les) Etat(s) contractant(s) suivant(s): ES, GR)

1. Procédé de préparation d'une composition pharmaceutique utile dans le traitement des troubles de la transmission cholinergique, comprenant l'utilisation comme ingrédient actif, d'un composé de formule I : 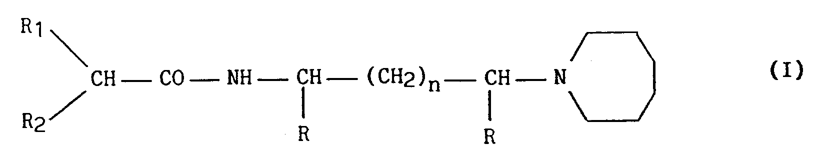 dans laquelle :
- R représente un atome d'hydrogène ou un radical alkyle de 1 à 4 atomes de carbone,
- n représente 0, 1, 2, 3, ou 4,
- R₁ et R₂, identiques ou différents représentent :
. un groupement alryle ou alkényle renfermant de 1 à 6 atomes de carbone, en chaine droite ou ramifiée,
. un groupement cycloalkyle comportant de 3 à 7 atomes de carbone ou cycloalkényle comportant de 5 à 7 atomes de carbone,
. un radical aryle, aralkyle, hétéroaryle ou hétéroaralkyle éventuellement substitué pour un ou plusieurs groupements choisis parmi halogène, alkoxy, nitro, trifluorométhyle ou hydroxyalkyle, étant entendu que par aryle on désigne les radicaux phényle ou naphtyle et par hétéroaryle on désigne les radicaux furyle, thiényle, pyrrolyle, imidazolyle, benzofuryle, benzothiényle, benzimidazolyle, indolyle,
leurs isomères ou de leurs sels d'addition à un acide pharmaceutiquement acceptable.

2. Procédé de préparation d'une composition pharmaceutique selon la revendication 1, comprenant l'utilisation comme ingrédient actif, du N-[3-(1-hexahydroazépinyl) propyl] diphénylacétamide, de l'un de ses isomères ou de l'un de ses sels d'addition à un acide pharmaceutiquement acceptable.

3. Procédé de préparation d'une composition pharmaceutique selon la revendication 1, comprenant l'utilisation comme ingrédient actif du N-[2-(1-hexahydroazépinyl) éthyl] diphénylacétamide, de l'un de ses isomères ou de l'un de ses sels d'addition à un acide pharmaceutiquement acceptable.

4. Procédé de préparation d'une composition pharmaceutique selon la revendication 1, comprenant l'utilisation comme ingrédient actif du cyclohexyl N-[3- (1-hexahydroazépinyl) propyl] thién-3 ylacétamide, de l'un de ses isomères ou de l'un de ses sels d'addition à un acide pharmaceutiquement acceptable.

5. Procédé de préparation d'une composition pharmaceutique selon la revendication 1, comprenant l'utilisation comme ingrédient actif du cyclohexyl N-[3- (1-hexahydroazépinyl) propyl] benzo [b] thién - 2 ylacétamide, de l'un de ses isomères ou de l'un de ses sels d'addition à un acide pharmaceutiquement acceptable.

6. Procédé de préparation d'une composition pharmaceutique selon l'une des revendications 1 à 5 destinée au traitement de la maladie d'Alzheimer, des désordres cérébraux liés à la sénescence ou des myasthénies.

7. Procédé de préparation d'une composition pharmaceutique selon la revendication 1 contenant l'ingrédient actif à la dose de 0,05 à 150 mg.

## Claims (Claims for the following Contracting State(s): AT, BE, CH, DE, DK, FR, GB, IT, LI, LU, NL, SE)

1. Use of a compound of formula I : 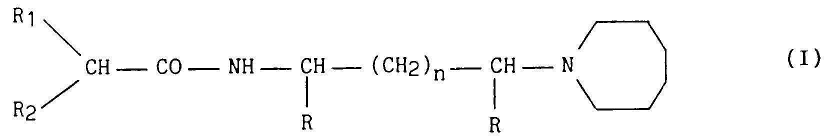 in which :
- R represents a hydrogen atom or an alkyl group containing from 1 to 4 carbon atoms,
- n represents 0, 1, 2, 3 or 4,
- R₁ and R₂, which are the same or different, represent:
. a straight-chain or branched alkyl or alkenyl group containing from 1 to 6 carbon atoms,
. a cycloalkyl group containing from 3 to 7 carbon atoms or a cycloalkenyl group containing from 5 to 7 carbon atoms,
. an aryl, aralkyl, heteroaryl or heteroaralkyl group optionally substituted by one or several groups selected from halogen, alkoxy, nitro, trifluoromethyl and hydroxyalkyl, aryl designating a phenyl or naphthyl group, and heteroaryl designating a furyl, thienyl, pyrrolyl, imidazolyl, benzofuryl, benzothienyl, benzimidazolyl or indolyl group,
one of its isomers, or one of its pharmaceutically acceptable acid addition salts, for obtaining drugs useful for the treatment of cholinergic transmission disorders.

2. Use according to claim 1, of N-[3-(1-hexahydroazepinyl) propyl]- diphenylacetamide, an isomer thereof or a pharmaceutically acceptable acid addition salt thereof for obtaining drugs useful for the treatment of cholinergic transmission disorders.

3. Use according to claim 1, of N-[2-(1-hexahydroazepinyl) ethyl]- diphenylacetamide, an isomer thereof or a pharmaceutically acceptable acid addition salt thereof for obtaining drugs useful for the treatment of cholinergic transmission disorders.

4. Use according to claim 1, of cyclohexyl N-[3-(1-hexahydroazepinyl) propyl]-thien-3-ylacetamide, an isomer thereof or a pharmaceutically acceptable acid addition salt thereof for obtaining drugs useful for the treatment of cholinergic transmission disorders.

5. Use according to claim 1, of cyclohexyl N-[3-(1-hexahydroazepinyl) propyl]-benzo[b]thien-2-ylacetamide, an isomer thereof or a pharmaceutically acceptable acid addition salt thereof for obtaining drugs useful for the treatment of cholinergic transmission disorders

6. Use according to any one of claims 1 to 5, for obtaining drugs useful for the treatment of the Alzheimer's disease, cerebral disorders associated with senescence, or myasthenias.

## Claims (Claims for the following Contracting State(s): ES, GR)

1. Process for preparing a pharmaceutical composition useful for the treatment of cholinergic transmission disorders, comprising the use, as active ingredient, of a compound of formula I : 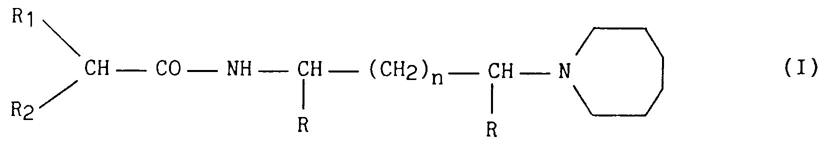 in which :
- R represents a hydrogen atom or an alkyl group containing from 1 to 4 carbon atoms,
- n represents 0, 1, 2, 3 or 4,
- R₁ and R₂, which are the same or different, represent:
. a straight-chain or branched alkyl or alkenyl group containing from 1 to 6 carbon atoms,
. a cycloalkyl group containing from 3 to 7 carbon atoms or a cycloalkenyl group containing from 5 to 7 carbon atoms,
. an aryl, aralkyl, heteroaryl or heteroaralkyl group optionally substituted by one or several groups selected from halogen, alkoxy, nitro, trifluoromethyl and hydroxyalkyl, aryl designating a phenyl or naphthyl group, and heteroaryl designating a furyl, thienyl, pyrrolyl, imidazolyl, benzofuryl, benzothienyl, benzimidazolyl or indolyl group,
its isomers or its pharmaceutically acceptable acid addition salts.

2. Process for preparing a pharmaceutical composition according to claim 1, comprising the use, as active ingredient, of N-[3-(1-hexahydroazepinyl) propyl]-diphenylacetamide, an isomer thereof or a pharmaceutically acceptable acid addition salt thereof.

3. Process for preparing a pharmaceutical composition according to claim 1, comprising the use, as active ingredient, of N-[2-(1-hexahydroazepinyl) ethyl]-diphenylacetamide, an isomer thereof or a pharmaceutically acceptable acid addition salt thereof

4. Process for preparing a pharmaceutical composition according to claim 1, comprising the use, as active ingredient, of cyclohexyl N-[3-(1-hexahydroazepinyl) propyl]- thien-3-ylacetamide, an isomer thereof or a pharmaceutically acceptable acid addition salt thereof.

5. Process for preparing a pharmaceutical composition according to claim 1, comprising the use, as active ingredient, of cyclohexyl N-[3-(1-hexahydroazepinyl) propyl]-benzo[b]thien-2-ylacetamide, an isomer thereof or a pharmaceutically acceptable acid addition salt thereof.

6. Process for preparing a pharmaceutical composition according to any one of claims 1 to 5, for the treatment of the Alzheimer's disease, cerebral disorders associated with senescence, or myasthenias.

7. Process for preparing a pharmaceutical composition according to claim 1, wherein the active ingredient is contained at a dose of from 0.05 mg to 150 mg.

## Patentansprüche (Patentansprüche für folgende(n) Vertragsstaat(en): AT, BE, CH, DE, DK, FR, GB, IT, LI, LU, NL, SE)

1. Verwendung einer Verbindung der Formel I: 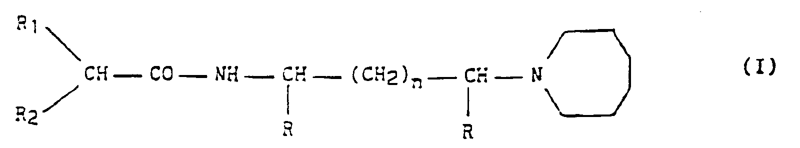 worin:
- R ein Wasserstoffatom oder einen Alkylrest mit 1 bis 4 Kohlenstoffatomen darstellt,
- n 0, 1, 2, 3 oder 4 darstellt,
- R₁ und R₂, die gleich oder verschieden sind, darstellen:
eine Alkyl- oder Alkenylgruppe, die 1 bis 6 Kohlenstoffatome enthält, mit gerader oder verzweigter Kette,
eine Cycloalkylgruppe, die 3 bis 7 Kohlenstoffatome umfaßt, oder eine Cycloalkenylgruppe, die 5 bis 7 Kohlenstoffatome umfaßt,
einen Aryl-, Aralkyl-, Heteroaryl- oder Heteroaralkylrest, der gegebenenfalls mit einer oder mehreren Gruppen, gewählt aus Halogen, Alkoxy, Nitro, Trifluormethyl oder Hydroxyalkyl, substituiert ist, wobei mit Aryl Phenyl- oder Naphthyl-Reste bezeichnet werden und mit Heteroaryl die Reste Furyl, Thienyl, Pyrrolyl, Imidazolyl, Benzofuryl, Benzothienyl, Benzimidazolyl und Indolyl bezeichnet werden,
eines ihrer Isomeren sowie eines ihrer Additionssalze mit einer pharmazeutisch annehmbaren Säure zur Herstellung von Arzneimitteln, die bei der Behandlung von Störungen des cholinergen Transmittersystems brauchbar sind.

2. Verwendung nach Anspruch 1 von N-[3-(1-hexahydroazepinyl)propyl]diphenylacetamid, einem seiner Isomeren oder einem seiner Additionssalze mit einer pharmazeutisch annehmbaren Säure zur Herstellung von Arzneimitteln, die bei der Behandlung von Störungen des cholinergen Transmittersystems brauchbar sind.

3. Verwendung nach Anspruch 1 von N-[2-(1-hexahydroazepinyl)ethyl]diphenylacetamid, einem seiner Isomeren oder einem seiner Additionssalze mit einer pharmazeutisch annehmbaren Säure zur Herstellung von Arzneimitteln, die bei der Behandlung von Störungen des cholinergen Transmittersystems brauchbar sind.

4. Verwendung nach Anspruch 1 von Cyclohexyl-N-[3-(1-hexahydroazepinyl)propyl]thien-3-yl-acetamid, einem seiner Isomeren oder einem seiner Additionssalze mit einer pharmazeutisch annehmbaren Säure zur Herstellung von Arzneimitteln, die bei der Behandlung von Störungen des cholinergen Transmittersystems brauchbar sind.

5. Verwendung nach Anspruch 1 von Cyclohexyl-N-[3-(1-hexahydroazepinyl)propyl]benzo[b]thien-2-yl-acetamid, einem seiner Isomeren oder einem seiner Additionssalze mit einer pharmazeutisch annehmbaren Säure zur Herstellung von Arzneimitteln, die bei der Behandlung von Störungen des cholinergen Transmittersystems brauchbar sind.

6. Verwendung einer Verbindung nach einem der Ansprüche 1 bis 5 zur Herstellung von Arzneimitteln, die bei der Behandlung der Alzheimer-Erkrankung, von altersbedingten cerebralen Störungen oder Myasthenien brauchbar sind.

## Patentansprüche (Patentansprüche für folgende(n) Vertragsstaat(en): ES, GR)

1. Verfahren zur Herstellung einer pharmazeutischen Zusammensetzung, die bei der Behandlung von Störungen des cholinergen Transmittersystems brauchbar ist, umfassend die Verwendung einer Verbindung der Formel I: 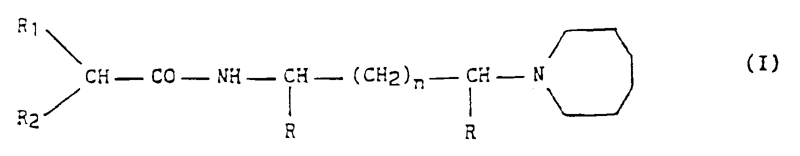 worin:
- R ein Wasserstoffatom oder einen Alkylrest mit 1 bis 4 Kohlenstoffatomen darstellt,
- n 0, 1, 2, 3 oder 4 darstellt,
- R₁ und R₂, die gleich oder verschieden sind, darstellen:
eine Alkyl- oder Alkenylgruppe, die 1 bis 6 Kohlenstoffatome enthält, mit gerader oder verzweigter Kette,
eine Cycloalkylgruppe, die 3 bis 7 Kohlenstoffatome umfaßt, oder eine Cycloalkenylgruppe, die 5 bis 7 Kohlenstoffatome umfaßt,
einen Aryl-, Aralkyl-, Heteroaryl- oder Heteroaralkylrest, der gegebenenfalls mit einer oder mehreren Gruppen, gewählt aus Halogen, Alkoxy, Nitro, Trifluormethyl oder Hydroxyalkyl, substituiert ist, wobei mit Aryl Phenyl- oder Naphthyl-Reste bezeichnet werden und mit Heteroaryl die Reste Furyl, Thienyl, Pyrrolyl, Imidazolyl, Benzofuryl, Benzothienyl, Benzimidazolyl und Indolyl bezeichnet werden,
ihrer Isomeren oder ihrer Additionssalze mit einer pharmazeutisch annehmbaren Säure als Wirkstoff.

2. Verfahren zur Herstellung einer pharmazeutischen Zusammensetzung nach Anspruch 1, umfassend die Verwendung von N-[3-(1-hexahydroazepinyl)propyl]diphenylacetamid, einem seiner Isomeren oder einem seiner Additionssalze mit einer pharmazeutisch annehmbaren Säure als Wirkstoff.

3. Verfahren zur Herstellung einer pharmazeutischen Zusammensetzung nach Anspruch 1, umfassend die Verwendung von N-[2-(1-hexahydroazepinyl)ethyl]diphenylacetamid, einem seiner Isomeren oder einem seiner Additionssalze mit einer pharmazeutisch annehmbaren Säure als Wirkstoff.

4. Verfahren zur Herstellung einer pharmazeutischen Zusammensetzung nach Anspruch 1, umfassend die Verwendung von Cyclohexyl-N-[3-(1-hexahydroazepinyl)propyl]thien-3-yl-acetamid, einem seiner Isomeren oder einem seiner Additionssalze mit einer pharmazeutisch annehmbaren Säure als Wirkstoff.

5. Verfahren zur Herstellung einer pharmazeutischen Zusammensetzung nach Anspruch 1, umfassend die Verwendung von Cyclohexyl-N-[3-(1-hexahydroazepinyl)propyl]benzo[b]thien-2-yl-acetamid, einem seiner Isomeren oder einem seiner Additionssalze mit einer pharmazeutisch annehmbaren Säure als Wirkstoff.

6. Verfahren zur Herstellung einer pharmazeutischen Zusammensetzung nach einem der Ansprüche 1 bis 5, die zur Behandlung der Alzheimer-Erkrankung, von altersbedingten cerebralen Störungen oder Myasthenien bestimmt ist.

7. Verfahren zur Herstellung einer pharmazeutischen Zusammensetzung nach Anspruch 1, welche den Wirkstoff in einer Dosis von 0,05 bis 150 mg enthält.
